# EUROPEAN PATENT SPECIFICATION

(11) **EP 4 362 861 B1**
(45) Date of publication and mention of the grant of the patent: **05.08.2026**
(21) Application number: 21763157.1
(22) Date of filing: 30.06.2021
(51) Int. Cl.: A61F 2/82, A61F 2/95, A61F 2/06, A61B 17/11, A61F 2/07, A61F 2/915, A61F 2/966

(54) **A SYSTEM FOR CONNECTING A BIONIC ORGAN TO A VASCULAR GRAFT AND A METHOD OF CONNECTING A BIONIC ORGAN TO A VASCULAR GRAFT**
SYSTEM ZUR VERBINDUNG EINES BIONISCHEN ORGANS MIT EINEM GEFÄSSTRANSPLANTAT UND VERFAHREN ZUR VERBINDUNG EINES BIONISCHEN ORGANS MIT EINEM GEFÄSSTRANSPLANTAT
SYSTÈME DE RACCORDEMENT D'UN ORGANE BIONIQUE À UN GREFFON VASCULAIRE ET PROCÉDÉ DE RACCORDEMENT D'UN ORGANE BIONIQUE À UN GREFFON VASCULAIRE

(43) Date of publication of application: 08.05.2024
(73) Proprietor: Polbionica S.A., 66-002 Zielona Góra (PL)
(72) Inventor: WSZOLA, Michal, 03-532 Warszawa (PL); KLAK, Marta, 64-920 Pila (PL); BERMAN, Andrzej, 00-565 Warszawa (PL); BRYNIARSKI, Tomasz, 34-400 Nowy Targ (PL); DOBRZANSKI, Tomasz, 02-987 Warszawa (PL)
(74) Representative: AOMB Polska Sp. z.o.o.
(86) International application number: PCT/PL2021/050050
(87) International publication number: WO 2023/277714

(56) References cited:
- EP-A1- 2 146 674
- US-A1- 2008 132 923
- US-A1- 2010 262 171
- US-A1- 2012 271 400
- US-A1- 2012 277 774
- US-B2- 8 491 612

## Description

### Field of invention

This invention relates to a system for connecting a bionic organ to a vascular graft and a method for connecting a bionic organ to a vascular graft under *ex vivo* conditions. Once connected, the vascular graft is situated within the bionic organ, and a stent is situated internally to the vascular graft and the bionic organ.

### Prior Art

Various types of stents are known from the prior art. Stents for medical applications are predominantly designed to dilate blood vessels following stenosis thereof. An example of such a stent is described in European patent EP1608299, which presents a self-expanding or balloon-expandable stent whose expansion within a blood vessel can occur simultaneously from both ends of the stent until the stent becomes fully expanded. The stent described herein is further provided with markers in the actuating part, which allow for the device to be located inside a human body. A tip of the stent was termed as non-damaging.

Another document regarding stents, European patent application EP3160400, presents a stent for use in a human body, comprising a flexible tip that facilitates insertion into a target site, loops in a connecting part to securely fix the stent and X-ray markers used to assess a position of the stent. The stent provides for a connection that allows for fluids to flow between a recipient body site into which the stent had been inserted and another end of the stent. Such stent is not self-expanding and is connected to a catheter in order to supply fluids to or drain them from the body. Its use in the human body is temporary.

European patent application EP2146674, in turn, describes a self-expanding stent that is inserted into a blood vessel using a special mandrel. Such a mandrel has a sharpened tip which, when the former is inserted into a target site, breaks open to allow the stent to exit through said tip. The mandrel is then withdrawn, and the stent is expanded and remains in the target site.

A similar solution is presented in another patent, JP4857125, wherein again a stent is delivered to a target site in a mandrel that is equipped with a longitudinally breaking tip. Once inserted, the tip breaks and provides sufficient lumen for the stent to pass through it. The stent then self-expands, which keeps it in the target site.

A possibility was also described in the prior art of connecting blood vessels together using stents, for example as indicated in US patent US9241782. The document describes a use of a self-expanding or balloon-expandable stent for connecting two blood vessels together. Such a connection may further comprise using expanding rings and clamps at both ends of the stent designed to securely fix the vessels to the stent. The stent may be present on the outside of the vessels or on the inside of the vessels. Stent-vessel connection is carried out as follows (for a stent on the outside of the vessel): 1) the stent in a compressed state is placed between the vessels that are spaced apart, which the stent is intended to connect, and the stent is expanded so that its diameter is greater than that of the vessels; 2) the vessels are placed in the stent; 3) the stent is contracted so that its diameter is smaller than the diameter of the expanded stent but larger than the diameter of the stent the compressed state; 4) the stent may be secured onto the vessels using expanding rings or micro-clamping stents (placed inside the vessels) so that the vessels are clamped between the stent and the ring at both ends of the stent. A plurality of such rings or other corresponding fixtures (flanges, clamps, etc.) may be used, but not necessarily. If a stent is used on the inside of the vessels, assembly process is similar, with rings (clamps) placed on the outer sides of the vessels. A mixed configuration is also possible, where one end of the stent is situated in the first vessel and the other end of the stent surrounds the second vessel.

Another US patent, US6336937, describes a possibility of using a self-expanding stent to bypass an embolic fragment of a blood vessel. In such a bypass, two expandable stent tips are placed in the blood vessel upstream and downstream of a blockage site, respectively, and are connected with a flexible tube (e.g. made of polymer). The length of the tube is selected as needed so as to provide a by-pass. In order to place the device in a blood vessel, the latter is incised where appropriate, and the tip of the stent is inserted inside.

The tip will expand, which will keep it fixed inside the vessel. Through the tube, blood will flow to another end of the bypass connection, placed in a similar manner in the blood vessel at the site behind the blockage.

US patent application US20200360126A1 describes a self-expanding or balloon-expandable stent used at a junction site of two vessels to prevent vessel stenosis following anastomosis. In order to perform the anastomosis, the vessels are connected by means of a suture, clip or another element used to connect the vessels. In this solution, the stent supports the structure and ensures that the vessels are properly fixed in relation to each other. The stent further prevents restenosis of the blood vessels. The blood vessels to be connected may have different diameters, so that the stent to be inserted thereto also needs to have a different diameter after it expands at either end. A change in diameter may be gradual along a length of the stent or it may be abrupt at a particular point of the stent.

US20120271400A1 describes an introducer system for repairing body vessels, particularly for emergency open surgical procedures or intraoperative repair. The system includes a prosthesis with a first and second retaining member to facilitate insertion and expansion of the prosthesis within the vessel.

### Object of the invention

The object of the invention is to develop a method for reliably and permanently connecting a bionic organ and a vascular graft together outside a living organism under *ex vivo* conditions. Said object was achieved by employing a system with a design suitable for this use.

### Description of the invention

The invention relates to a system for connecting a bionic organ to a vascular graft which comprises a self-expanding stent, a casing and a mandrel with a breakable tip, wherein, in an assembled state of the system, the stent in a compressed state is placed in the casing, which casing holds the stent in a compressed state until the stent is removed from the casing, and the mandrel is placed inside the stent for removing the stent from the casing, wherein, in the assembled state, the length of the casing and the mandrel is in the range of 20 to 40 cm, the length of the stent is in the range of 10 to 40 mm, and the diameter of the stent is selected such that, in the compressed state, the diameter of the stent is less than diameter of the vascular graft and, when expanded, the diameter of the stent is greater than or equal to diameter of the vascular port in the bionic organ.

Preferably, in the expanded state, the diameter of the stent ranges from 0.2 mm to 50 mm, preferably from 1 to 20 mm.

In the assembled state, the casing and mandrel may have a length of 30 cm, and the stent may have a length of 20 mm.

Preferably, the mandrel comprises a circumferential notch that allows the breakable end of the mandrel to break off.

The breakable tip of the mandrel may be conical with a rounded end.

Preferably, the stent comprises at least one fixing loop, preferably it comprises two fixing loops, one at each end of the stent.

Preferably, the casing comprises a depth indicator.

The invention also relates to a method for connecting a bionic organ to a vascular graft *ex vivo,* wherein the connection is achieved by means of a system of the invention, which method comprises the following steps:
a) Inserting the system in the assembled state into the vascular graft
b) Partially removing the stent from the casing and attaching the stent to the vascular graft, preferably by means of a loop
c) Breaking off the tip of the mandrel, preferably at a circumferentially notched point
d) Inserting the vascular graft with the attached stent in the casing into the vascular port of the bionic organ
e) Removing the stent from the casing by using the mandrel with the broken off tip
f) Removing the casing and the mandrel with the broken off tip from the vascular port of the bionic organ.

They following may be used as the vascular graft:
- a decellularized or recellularized vessel of animal origin, or
- a preserved vessel of animal origin, or
- an autogenous vessel, preferably a saphenous vein, or
- a vessel harvested from a deceased donor and preserved, or
- a vessel harvested from a deceased donor and non-preserved, or
- a vascular prosthesis, preferably made of EPTFE (expanded polytetrafluoroethylene).

Prior to implementing the step a), the vascular graft may be decellularized and then populated with endothelial cells.

The bionic organ may be made using 3D bioprinting technology.

Preferably, the insertion depth in step d) is regulated by means of a depth indicator and corresponds to the length of the stent to be used.

Preferably, in step d) the stent is inserted to a depth of 10 to 40 mm, more preferably 15 to 30 mm, and most preferably to a depth of 20 mm.

Most preferably, the sequence of steps a) to f) is performed for each vascular port of the bionic organ.

### Brief description of figures

The object of the invention in the embodiment is shown in the drawing, where:
fig. 1 shows a stent 2 in its compressed state prior to its insertion in a vascular graft 4, where: 1 is a mandrel of the stent, and 3 is a casing of the stent;
fig. 2 shows the stent 2 after it is fixed in the vascular graft 4 and before it is installed in a vascular port 7 inside a bionic organ 8, where 1* is the mandrel after the tip has been broken off, 5 is a depth indicator, and 6 is a fixing loop;
fig. 3 shows the stent 2 connected to the vascular graft 4 after it is installed in the vascular port 7;
fig. 4 shows a set of samplers with different diameters for selecting the diameter of the stent 2 for the vascular graft 4;
fig. 5 shows the bionic organ 8, an artificial pancreas, connected through its vascular port 7 to the vascular graft 4 by means of the stent 2, and a flow of contrast medium across the bionic organ 8.

For the purposes of the present invention, a vascular graft is defined as any blood vessel of animal origin, such as for example a blood vessel harvested from a pig, cow, sheep and other animals, or of human origin, previously harvested from the human body and not connected with the human or animal body as part of implementation of the method according to the invention.

Preferably, such a vessel is previously decellularized, i.e. it is stripped of cells and foreign DNA in order to eliminate a possibility of tissue incompatibility with a potential recipient. Even more preferably, following decellularization, such a vessel is then populated with recipient cells. Decellularization may be achieved using any method known in the art, such as for example a flow method or using a static system where the vessels are in a detergent solution in a vessel placed on a shaker.

The term "bionic organ" or "artificial organ" denotes an artificially obtained three-dimensional structure that imitates any organ, such as pancreas, lung, heart, liver, etc. Preferably, the bionic organ is obtained using 3D bioprinting. The bionic organ comprises a vascular system, ending with vascular ports.

The method according to the invention provides a fast, tight and permanent connection of the bionic organ and the vascular graft.

The vascular graft thus connected together with the bionic organ may then be connected to recipient's vascular system, such as by means of a vascular suture (indirect connection using a vascular graft).

The connection method according to the invention may also be used to directly connect the bionic organ to the recipient's vascular system by directly connecting the organ to the recipient's vessel using an *in situ* stent. More specifically, such a connection is performed by making an incision on the recipient's vessel upstream of the connection point with the bionic organ and inserting a stent thereto in order to expand it and to secure the connection. The following options are possible for connecting the bionic organ to the recipient's vascular system *in situ:*
- isolated arterial and venous vessels of the recipient *in situ -* here, steps a) to c) of the method according to the invention are replaced as follows: terminal vessels of the recipient (cut at one end) (e.g. epigastric vein and artery, internal iliac vein and artery) isolated over a distance of at least 30 mm are temporarily closed, preferably with a vascular clamp, and an incision is made therein near to the closure site, so as to introduce the system according to the invention through the opening provided towards the cut end.

Further steps of connecting the vessel to the vascular port of the bionic organ are as in the *ex vivo* method according to the invention. Once the mandrel is removed, the opening in the vessel is closed with a vascular suture.
- isolated arterial and venous vessels of the recipient *in situ* - here, steps a) to c) of the method according to the invention are replaced as follows: terminal vessels of the recipient (e.g. epigastric vein and artery, internal iliac vein and artery) isolated over a distance of at least 40 mm are temporarily closed, preferably with a vascular clamp, and an incision is made therein in the middle. An incision is then made in the vessel, preferably in the vicinity of both clamps, so as to introduce the systems according to the invention through the openings obtained towards the cut-off ends. The further steps of connecting the vessels to the vascular ports of the bionic organ (which, here, has vascular ports arranged at opposite ends) are as for the *ex vivo* method according to the invention. Once the mandrels are removed, the openings in the vessels are closed with a vascular suture.

The system for connecting a bionic organ to a vascular graft has undergone various modifications with respect to a commercially available stent in order to allow for it to be used in the method according to the invention.

The stent described in the present application is housed in a casing that keeps it in a compressed (retracted) state until it is removed from the casing, and it comprises a mandrel to allow it to be removed from the casing. Once the stent is removed from the casing, it expands and assumes the expanded state.

Such a stent may further comprise a depth indicator on its casing to allow for accurate positioning thereof during insertion, in particular during insertion into the vascular system of the bionic organ. Said depth indicator allows for precision embedding of the stent with the vascular graft in the inlet/outlet opening (vascular port) of the bionic organ so as to optimise insertion.

The stent described in this application may be provided with loops provided at each end thereof that extend beyond the casing of the stent. Said loops are used to fix the stent to the vascular graft using surgical thread. Relative to commercially available systems, the system disclosed in the present application is characterised by a reduced length of the casing and mandrel in the assembled state. Said length ranges from 20 to 40 cm, preferably equals 30 cm. The length thus reduced facilitates handling of the stent under laminar chamber conditions.

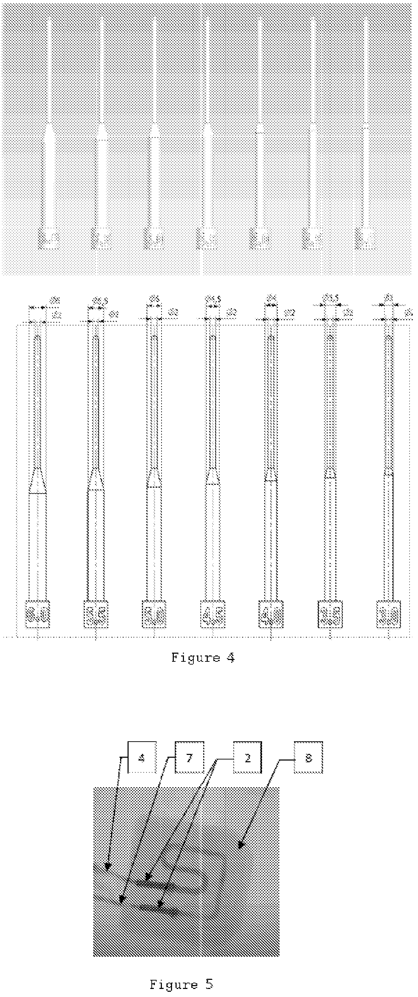

The stent is also available in a variety of diameter ranges and lengths of the stent itself. The diameters of the stent when fully expanded, wherein full expansion is meant to be the maximum expansion of the stent outside the vascular graft and the vascular port, are in the range of 0.2 to 50 mm, preferably 1 to 20 mm, and are suitably selected so that the diameter of the stent prior to expansion is less than that of the vascular graft and that the stent expands to a diameter greater than or equal to that of the vascular port. Preferably, the diameter of the stent after expanding is between 100% and 150% of the diameter of the vascular port. Examples of stents with various diameters are shown in Fig. 4. The length of the stent, in turn, ranges from 10 to 40 mm, preferably equals 20 mm. Such a range of available stent diameters and lengths provides extensive adaptation possibilities depending on a type of organ to be implanted and the diameter of the vascular graft and vascular port.

Furthermore, the system according to the present invention comprises a mandrel with a breakable tip. Said mandrel may be provided with a notch along its circumference at the level of the outer end of the stent, which allows the tip of the mandrel to break off in the determined place. The tip of the mandrel being breakable prevents damage to the bionic organ or vascular port during the insertion of the vascular graft thereto together with the stent. This solution also eliminates a need for the vessels in bionic organs to be designed in straight line with respect to the vascular ports. Breaking off of the tip prevents the mandrel from abutting against the vessel wall of the bionic organ when the stent is being pushed out, so that it does not press on the vessel wall.

The modifications of existing solutions as above make the system according to the invention a unique and currently unavailable product, that may play a crucial part in the field of transplantation, in particular in cases of transplantation of bionic organs.

Preferably, the system according to the invention comprises a set of samplers that allow to accurately tailor the diameter of the stent to the vascular graft. Such a set of samplers may include the following samplers with diameters from 0.2 to 50 mm, preferably from 1 to 20 mm, wherein the set preferably comprises samplers in the full range of diameters from 1 to 20 mm with 0.5 mm increments.

### Embodiments

### Example 1 - Preparation of a stent for use in the method according to the invention based on a commercial stent

In order to obtain a stent (2) suitable for use in the method according to the invention, the following modifications have been made relative to a commercially available stent with size of 6 mm x 20 mm:
- Shortening of the mandrel (1) and casing (3) set of the stent (2) to a length of 30 cm so that it can be handled *ex vivo* in a laminar chamber. For comparison, the length of commercial mandrel-casing sets is, for example, 125 cm;
- Making a notch on the casing (3) of the stent (2) with a length of approximately 5 mm to allow subsequent placement of a surgical thread on the first loop of the stent without opening the stent;
- Creating two fixing loops (6) on the outer edge of the stent (2), one at each end of the stent (2);
- Creating a circumferential notch on the mandrel (1) in the proximity of the point where it retracts in the casing (3), so as to facilitate its subsequent breaking or twisting;
- Creating a depth indicator (5) on the casing (3) of the stent (2).

### Example 2 - Implementation of the connection between vascular graft and artificial pancreas (ex vivo stage of artificial pancreas transplantation)

### 1) Creation of a vascular graft

Pig splenic arteries obtained from a local abattoir were used to create the graft (4). Using gauges, vessels with a diameter of 3.5 to 4 mm and a length of approximately 60 mm were selected. The vessels were then subjected to flow decellularization in a closed system at a constant flow rate of 40 mL/min. The decellularization process consisted of the following steps:
- 48h flow of the solution of 1% Triton X-100 + 0.1% NH4OH in 1xPBS at a temperature of 4 °C,
- 48h flow of 1xPBS solution with 0.01% streptomycin at a temperature of 4 °C,
- 8h flow of 1xPBS solution with 0.0002% DNase I and 0.12mM Ca²+ and Mg²+ at a temperature of 37 °C,
- 48h flow of 1xPBS solution with 0.01% streptomycin at a temperature of 4 °C.

The vessels were then preserved in 1xPBS solution with 0.01% streptomycin and subjected to radiation sterilisation at a radiation dose of 25 kGy. Finally, the vessels were repopulated with recipient's cells (using recipient's endothelial cells).

### 2) Stent selection

A self-expanding stent (2) with a diameter of 6 mm and a length of 20 mm, obtained as described in example 1, was used in the experiment.

### 3) Artificial pancreas to be connected to a vascular graft

A bio-printed organ, an artificial pancreas (8) was used in the experiment. The organ (8) was obtained by means of extrusion bioprinting technology using a bioink, which is the subject of another patent application EP19218191.5 by the same applicants, which is still pending at the date of filing this application. Organ housing components (8) were printed using SLA technology with photo-curable polymers. Inlet and outlet - vascular ports (7) of the artificial pancreas (8) had identical diameters of 4 mm.

### 4) Course of experiment

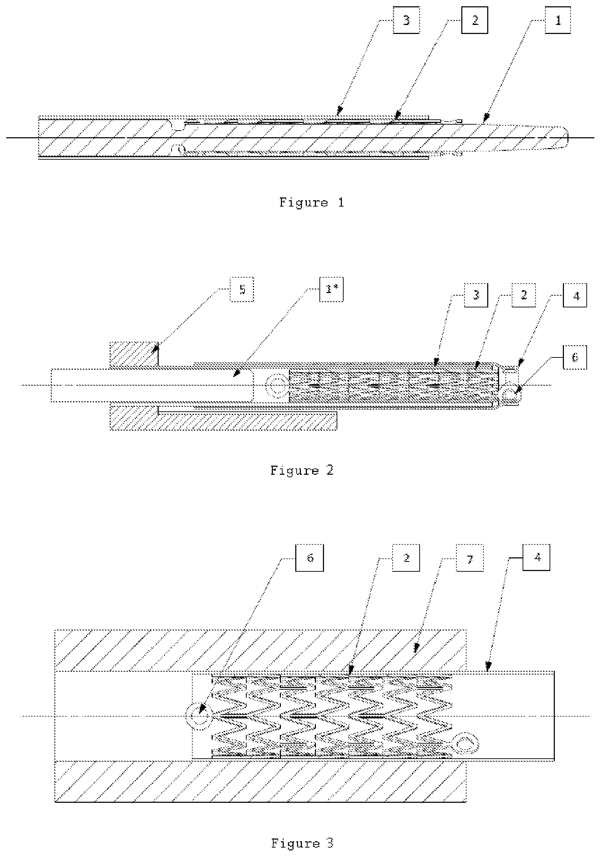

The method is schematically illustrated in Fig. 1-3. Fig. 1 shows the stent (2) in its compressed state prior to its inserting in the vascular graft (4). The stent (2) was in a compressed configuration and was situated inside the casing (3). The casing was equipped with a mandrel (1), which allowed simple and non-damaging insertion of the stent (2) into the vascular graft (4) thanks to its rounded, tapered tip.

In the first step of connecting the bionic organ (8) to the vascular graft (4), shown in Fig. 2, a stent (2) in a compressed configuration arranged in the casing (3) was inserted into the vascular graft (4) to the appropriate depth. A stent in the casing (3) was inserted into the vascular graft so that it was entirely located inside the graft.

Once the stent (2) together with the casing (3) was inserted into the vascular graft (4), the stent (2) was gently removed from the casing (3) so that it did not open completely to allow for fixing it to the vascular graft (4) via fixing loops (6) at both ends of the stent (2). This was achieved by suturing a loop (6) to the vascular graft (4) with surgical thread with the purpose to immobilise the stent (2) in the graft (4). The stent (2) was sutured to the vascular graft (4) using a single vascular suture (Prolen 4-0) at both ends of the stent (2). The tip of the mandrel (1) protruding beyond an outline of the casing (3) at a suitable, circumferentially notched point, was broken off. In this step, the tip has already fulfilled its function, i.e. it allowed for the vascular graft (4) to slide over the casing (3) and, more than being no longer necessary, it even hindered the execution of subsequent steps of the method. Two such structures connecting the vascular graft (4) to the stent (2) were prepared.

In the next step, a vascular graft (4) with a stent (2) sutured thereto in a casing (3) was inserted into the vascular port (7). The prepared structures connecting the vascular graft (4) to the stent (2) were inserted into the arterial and venous vascular ports (7) of the artificial pancreas (8), respectively, to a depth of 20 mm. This depth also corresponded to the length of the stent (2) used. The latter value was particularly important as it guaranteed proper fixing without undue enlargement of the entire organ. The inner diameter of the vascular graft (4) was selected such that it was 0.0-0.5mm larger than the inner diameter of the vascular port (7). In this step, the insertion depth was controlled using a depth indicator (5).

In the next step, respectively, each of the two self-expandable stents (2) was removed from the casing (3) using a mandrel (1*), which caused the stent (2) to expand to an expanded state.

The casing (3) and the mandrel (1*) of the stent (2) were then removed from both vascular ports (7) of the artificial pancreas (8), which resulted in the stent (2) expanding into the expanded state, shown in fig. 3, i.e. to a diameter corresponding to that of the inlet and outlet of the artificial pancreas (8), i.e. 4 mm, which in turn resulted in the expansion of the vascular graft (4) and immobilisation of the stents (2) together with the vascular grafts (4) in the lumen of the respective vascular ports (7). In this step, the connecting means was the expansion force of the stent (2), which ensured not only connection, but also fixation of the vascular grafts (4) in the vascular ports (7), while preventing stenosis along the length of the stent (2). Opening the stent (2) resulted in expansion of the proximal section of the stent (2) slightly (over a length of approximately 2 mm) beyond the contour of the vascular graft (4) being implanted. The exposed part of the stent (2) situated outside the graft (4) anchored the artificial pancreas (8) in the vascular port (7), and the further funnel-like tapering part sealed the vascular port (7).

### 5) Results

The artificial pancreas (8) obtained in this experiment is illustrated in fig. 5. The figure shows the flow of a contrast medium through the pancreas (8), from the inlet vascular graft to the outlet vascular graft. Fixing of vascular grafts (4) with a stent (2) is marked black.

*Ex vivo* tests performed demonstrated the tightness of the connection at a pressure up to 180 mmHg. Above this value, tightness deficits were observed in the artificial pancreas (8), but they only involved a dissection in the functional part of the bionic pancreas (8) rather than the vascular ports (7).

The artificial pancreas (8) obtained as described in example 2, connected to the vascular grafts (4), was implanted into the body of a live pig by making an incision in the respective pig iliac blood vessels and connecting them using end-to-side Carrel sutures to the vascular graft (4) located in venous and arterial vascular ports (7) of the artificial pancreas (8), respectively. After 14 days, the artificial pancreas (8) was again removed and dissected for visual inspection.

The results of the *in vivo* experiment confirmed that the connection between the vascular graft (4) and the artificial organ (8) thus obtained was durable and tight.

## Claims

1. A system for connecting a bionic organ to a vascular graft, **characterised in that** it comprises a self-expanding stent (2), a casing (3) and a mandrel (1) with a breakable tip, wherein in an assembled state of the system the stent (2) in a compressed state is placed in the casing (3), which casing (3) holds the stent (2) in a compressed state until the stent (2) is removed from the casing (3), and the mandrel (1) is placed inside the stent (2) for removing the stent (2) from the casing (3), wherein, in the assembled state, the length of the casing (3) and the mandrel (1) is in a range of 20 to 40 cm, the length of the stent (2) is in a range of 10 to 40 mm, and a diameter of the stent (2) is selected such that, in the compressed state, the diameter of the stent (2) is less than diameter of the vascular graft (4) and, when expanded, the diameter of the stent (2) is greater than or equal to diameter of the vascular port (7) in the bionic organ (8).

2. The system according to claim 1 **characterised in that** in the expanded state, the diameter of the stent (2) ranges from 0.2 mm to 50 mm, preferably from 1 to 20 mm.

3. The system according to claim 1 or 2, **characterised in that** in the assembled state, the casing (3) and the mandrel (1) have a length of 30 cm, and the stent (2) has a length of 20 mm.

4. The system according to claim 1 or 2 or 3, **characterised in that** the mandrel (1) comprises a circumferential notch which allows the breakable tip of the mandrel (1) to be broken off.

5. The system according to any one of claims 1 to 4, **characterised in that** the breakable tip of the mandrel (1) has a conical shape with a rounded end.

6. The system according to any one of claims 1 to 5, **characterised in that** the stent (2) comprises at least one fixing loop (6), preferably two fixing loops, one at each end of the stent (2).

7. The system according to any one of claims 1 to 6, **characterised in that** the casing (3) comprises a depth indicator (5).

8. A method for connecting a bionic organ to a vascular graft under *ex vivo* conditions, **characterised in that** the connection is achieved by means of a system as defined in any one of claims 1 to 7, wherein said method comprises the following steps:
a) Inserting the system in the assembled state into the vascular graft (4)
b) Partially removing the stent (2) from the casing (3) and attaching the stent (2) to the vascular graft (4), preferably by means of a loop (6)
c) Breaking off the tip of the mandrel (1), preferably at the circumferentially notched point
d) Inserting the vascular graft (4) with the attached stent (2) in the casing (3) into the vascular port (7) of the bionic organ (8)
e) Removing the stent (2) from the casing (3) using the mandrel with the broken off tip (1*)
f) Removing the casing (3) and the mandrel with the broken off tip (1*) from the vascular port (7) of the bionic organ (8).

9. The method according to claim 8, **characterised in that** the following are used as the vascular graft (4):
- a decellularized or recellularized vessel of animal origin, or
- a preserved vessel of animal origin, or
- an autogenous vessel, preferably a saphenous vein, or
- a vessel harvested from a deceased donor and preserved, or
- a vessel harvested from a deceased donor and non-preserved, or
- a vascular prosthesis, preferably made of EPTFE (expanded polytetrafluoroethylene)

10. The method according to claim 8 or 9, **characterised in that** prior to implementing step a), the vascular graft (4) is decellularized and then populated with endothelial cells.

11. The method according to any one of claims 8 to 10, **characterised in that** the bionic organ (8) is prepared using 3D bioprinting technology.

12. The method according to any one of claims 8 to 11, **characterised in that** insertion depth in step d) is controlled by the depth indicator (5) and it corresponds to the length of the stent (2) used.

13. The method according to claim 12, **characterised in that** in step d) the stent (2) is inserted to a depth of 10 to 40 mm, more preferably 15 to 30 mm, and most preferably to a depth of 20 mm.

14. The method according to any one of claims 8 to 13, **characterised in that** the sequence of steps a) to f) is performed for each of the vascular ports (7) of the bionic organ (8).

## Patentansprüche

1. System zum Verbinden eines bionischen Organs mit einem Gefäßtransplantat, **dadurch gekennzeichnet, dass** es einen selbstexpandierenden Stent (2), ein Gehäuse (3) und einen Dorn (1) mit einer abbrechbaren Spitze umfasst, wobei in einem zusammengesetzten Zustand des Systems der Stent (2) in einem komprimierten Zustand in dem Gehäuse (3) angeordnet ist, wobei das Gehäuse (3) den Stent (2) in einem komprimierten Zustand hält, bis der Stent (2) aus dem Gehäuse (3) entfernt wird, und der Dorn (1) innerhalb des Stents (2) zum Entfernen des Stents (2) aus dem Gehäuse (3) angeordnet ist, wobei im zusammengesetzten Zustand die Länge des Gehäuses (3) und des Dorns (1) in einem Bereich von 20 bis 40 cm liegt, die Länge des Stents (2) in einem Bereich von 10 bis 40 mm liegt und ein Durchmesser des Stents (2) so gewählt ist, dass im komprimierten Zustand der Durchmesser des Stents (2) kleiner als der Durchmesser des Gefäßtransplantats (4) ist und im expandierten Zustand der Durchmesser des Stents (2) größer oder gleich dem Durchmesser des Gefäßkatheters (7) in dem bionischen Organ (8) ist.

2. System nach Anspruch 1, **dadurch gekennzeichnet, dass** im expandierten Zustand der Durchmesser des Stents (2) im Bereich von 0,2 mm bis 50 mm, vorzugsweise von 1 bis 20 mm, liegt.

3. System nach Anspruch 1 oder 2, **dadurch gekennzeichnet, dass** im zusammengesetzten Zustand das Gehäuse (3) und der Dorn (1) eine Länge von 30 cm aufweisen und der Stent (2) eine Länge von 20 mm aufweist.

4. System nach Anspruch 1 oder 2 oder 3, **dadurch gekennzeichnet, dass** der Dorn (1) eine Kerbe in Umfangsrichtung aufweist, die es ermöglicht, die abbrechbare Spitze des Dorns (1) abzubrechen.

5. System nach irgendeinem der Ansprüche 1 bis 4, **dadurch gekennzeichnet, dass** die abbrechbare Spitze des Dorns (1) eine konische Form mit einem abgerundeten Ende aufweist.

6. System nach irgendeinem der Ansprüche 1 bis 5, **dadurch gekennzeichnet, dass** der Stent (2) mindestens eine Befestigungsschlaufe (6), vorzugsweise zwei Befestigungsschlaufen, eine an jedem Ende des Stents (2), aufweist.

7. System nach irgendeinem der Ansprüche 1 bis 6, **dadurch gekennzeichnet, dass** das Gehäuse (3) einen Tiefenanzeiger (5) umfasst.

8. Verfahren zum Verbinden eines bionischen Organs mit einem Gefäßtransplantat unter *ex vivo* Bedingungen, **dadurch gekennzeichnet, dass** die Verbindung mittels eines Systems, wie in irgendeinem der Ansprüche 1 bis 7 definiert, erreicht wird, wobei das Verfahren die folgenden Schritte umfasst:
a) Einsetzen des Systems im zusammengesetzten Zustand in das Gefäßtransplantat (4)
b) Teilweises Entfernen des Stents (2) aus dem Gehäuse (3) und Anbringen des Stents (2) am Gefäßtransplantat (4), vorzugsweise mittels einer Schlaufe (6)
c) Abbrechen der Spitze des Dorns (1), vorzugsweise an der in Umfangsrichtung gekerbten Stelle
d) Einsetzen des Gefäßtransplantats (4) mit dem angebrachten Stent (2) in das Gehäuse (3) in den Gefäßkatheter (7) des bionischen Organs (8)
e) Entfernen des Stents (2) aus dem Gehäuse (3) unter Verwendung des Dorns mit der abgebrochenen Spitze (1*)
f) Entfernen des Gehäuses (3) und des Dorns mit der abgebrochenen Spitze (1*) aus dem Gefäßkatheter (7) des bionischen Organs (8).

9. Verfahren nach Anspruch 8, **dadurch gekennzeichnet, dass** als Gefäßtransplantat (4) Folgendes verwendet wird:
- ein dezellularisiertes oder rezellularisiertes Gefäß tierischen Ursprungs, oder
- ein konserviertes Gefäß tierischen Ursprungs, oder
- ein körpereigenes Gefäß, vorzugsweise eine Saphenusvene, oder
- ein Gefäß, das einem verstorbenen Spender entnommen und konserviert wurde, oder
- ein Gefäß, das einem verstorbenen Spender entnommen und nicht konserviert wurde, oder
- eine Gefäßprothese, vorzugsweise aus EPTFE (expandiertes Polytetrafluorethylen)

10. Verfahren nach Anspruch 8 oder 9, **dadurch gekennzeichnet, dass** vor dem Implementieren von Schritt a) das Gefäßtransplantat (4) dezellularisiert und anschließend mit Endothelzellen besiedelt wird.

11. Verfahren nach irgendeinem der Ansprüche 8 bis 10, **dadurch gekennzeichnet, dass** das bionische Organ (8) unter Verwendung von 3D-Bioprinting-Technologie hergestellt wird.

12. Verfahren nach irgendeinem der Ansprüche 8 bis 11, **dadurch gekennzeichnet, dass** die Einführtiefe in Schritt d) durch den Tiefenanzeiger (5) gesteuert wird und sie der Länge des verwendeten Stents (2) entspricht.

13. Verfahren nach Anspruch 12, **dadurch gekennzeichnet, dass** in Schritt d) der Stent (2) bis zu einer Tiefe von 10 bis 40 mm, vorzugsweise 15 bis 30 mm, und am meisten bevorzugt bis zu einer Tiefe von 20 mm eingeführt wird.

14. Verfahren nach irgendeinem der Ansprüche 8 bis 13, **dadurch gekennzeichnet, dass** die Abfolge der Schritte a) bis f) für jeden der Gefäßkatheter (7) des bionischen Organs (8) durchgeführt wird.

## Revendications

1. Un système de connexion d'un organe bionique à un greffon vasculaire, **caractérisé en ce qu'**il comprend une endoprothèse auto-expansible (2), une enveloppe (3) et un mandrin (1) avec une pointe cassable, dans lequel, dans un état assemblé du système, l'endoprothèse (2) dans un état comprimé est placée dans l'enveloppe (3), l'enveloppe (3) maintient l'endoprothèse (2) dans un état comprimé jusqu'à ce que l'endoprothèse (2) soit retirée de l'enveloppe (3), et le mandrin (1) est placé à l'intérieur de l'endoprothèse (2) pour retirer l'endoprothèse (2) de l'enveloppe (3), dans lequel, à l'état assemblé, la longueur de l'enveloppe (3) et du mandrin (1) est comprise entre 20 et 40 cm, la longueur de l'endoprothèse (2) est comprise entre 10 et 40 mm, et le diamètre de l'endoprothèse (2) est choisi de manière à ce que, à l'état comprimé, le diamètre de l'endoprothèse (2) soit compris entre 10 et 40 mm, à l'état comprimé, le diamètre du stent (2) est inférieur au diamètre du greffon vasculaire (4) et, à l'état expansé, le diamètre du stent (2) est supérieur ou égal au diamètre de l'orifice vasculaire (7) dans l'organe bionique (8).

2. Le système selon la revendication 1, **caractérisé en ce qu'**à l'état expansé, le diamètre de l'endoprothèse (2) est compris entre 0,2 mm et 50 mm, de préférence entre 1 et 20 mm.

3. Le système selon la revendication 1 ou 2, **caractérisé en ce qu'**à l'état assemblé, l'enveloppe (3) et le mandrin (1) ont une longueur de 30 cm, et l'endoprothèse (2) a une longueur de 20 mm.

4. Le système selon la revendication 1 ou 2 ou 3, **caractérisé en ce que** le mandrin (1) comprend une encoche circonférentielle qui permet de casser la pointe cassable du mandrin (1).

5. Le système selon l'une des revendications 1 à 4, **caractérisé en ce que** la pointe cassable du mandrin (1) a une forme conique avec une extrémité arrondie.

6. Le système selon l'une des revendications 1 à 5, **caractérisé en ce que** l'endoprothèse (2) comprend au moins une boucle de fixation (6), de préférence deux boucles de fixation, une à chaque extrémité de l'endoprothèse (2).

7. Le système selon l'une des revendications 1 à 6, **caractérisé en ce que** l'enveloppe (3) comprend un indicateur de profondeur (5).

8. Un procédé de connexion d'un organe bionique à un greffon vasculaire dans des conditions *ex vivo ,* **caractérisé en ce que** la connexion est réalisée au moyen d'un système tel que défini dans l'une quelconque des revendications 1 à 7, dans lequel ledit procédé comprend les étapes suivantes :.
a) Insertion du système à l'état assemblé dans le greffon vasculaire (4)
b) Retrait partiel de l'endoprothèse (2) hors de l'enveloppe (3) et fixation de l'endoprothèse (2) au greffon vasculaire (4), de préférence au moyen d'une boucle (6).
c) Cassure de la pointe du mandrin (1), de préférence au niveau de l'encoche circonférentielle
d) Insertion du greffon vasculaire (4) avec l'endoprothèse (2) fixée dans l'enveloppe (3) dans l'orifice vasculaire (7) de l'organe bionique (8)
e) Retrait de l'endoprothèse (2) de l'enveloppe (3) à l'aide du mandrin avec la pointe cassée (1*)
f) Retrait du boîtier (3) et le mandrin avec la pointe cassée (1*) de l'orifice vasculaire (7) de l'organe bionique (8).

9. Le procédé selon la revendication 8, **caractérisé par** l'utilisation des éléments suivants comme greffon vasculaire (4) :
- un vaisseau décellularisé ou recellularisé d'origine animale, ou
- un vaisseau préservé d'origine animale, ou
- un vaisseau autogène, de préférence une veine saphène, ou
- un vaisseau prélevé sur un donneur décédé et préservé, ou
- un vaisseau prélevé sur un donneur décédé et non-préservé, ou
- une prothèse vasculaire, de préférence en EPTFE (polytétrafluoroéthylène expansé)

10. Le procédé selon la revendication 8 ou 9, **caractérisé en ce qu'**avant la mise en œuvre de l'étape a), le greffon vasculaire (4) est décellularisé, et puis peuplé de cellules endothéliales.

11. Le procédé selon l'une des revendications 8 à 10, **caractérisé en ce que** l'organe bionique (8) est préparé à l'aide de la technologie de bio-impression 3D.

12. Le procédé selon l'une des revendications 8 à 11, **caractérisé en ce que** la profondeur d'insertion à l'étape d) est contrôlée par l'indicateur de profondeur (5) et qu'elle correspond à la longueur de l'endoprothèse (2) utilisé.

13. Le procédé selon la revendication 12, **caractérisé en ce qu'**à l'étape d), l'endoprothèse (2) est insérée à une profondeur de 10 à 40 mm, de préférence de 15 à 30 mm, et de préférence encore à une profondeur de 20 mm.

14. Le procédé selon l'une des revendications 8 à 13, **caractérisé en ce que** la séquence des étapes a) à f) est réalisée pour chacun des orifices vasculaires (7) de l'organe bionique (8).
